# EUROPEAN PATENT APPLICATION

(11) **EP 3 441 020 A1**
(43) Date of publication of application: **13.02.2019**
(21) Application number: 18188275.4
(22) Date of filing: 09.08.2018
(51) Int. Cl.: A61B 17/128, A61B 90/92

(54) **CLIP APPLIERS WITH EXTENDED JAW TIP**

(30) Priority: 10.08.2017 US 201715674125
(71) Applicant: Ethicon LLC, Guaynabo 00969 (PR)
(72) Inventor: STOKES, Michael J., Cincinnati, OH 45242 (US); LAURENT, Ryan J., Cincinnati, OH 45242 (US); FEGELMAN, Elliott J., Cincinnati, OH 45242 (US); SCOTT, Gregory G., Cincinnati, OH 45242 (US); LABHASETWAR, Disha V., Cincinnati, OH 45242 (US)
(74) Representative: Bettridge, Paul Sebastian

(57) **Abstract**

A surgical clip applier and methods for applying surgical clips to a vessel, duct, shunt, etc., during a surgical procedure are provided. In one exemplary embodiment, a surgical clip applier is provided having an elongate shaft and first and second jaws on a distal end thereof. The first and second jaws are configured to move between open and closed positions for engaging tissue therebetween. Each jaw can have a clip engaging portion and the first jaw can have an extended tip portion that extends distally beyond the clip engaging portion such that the first jaw has a length that is greater than a length of the second jaw. The surgical clip applier can also include a clip advancing assembly that extends through the elongate shaft and that is configured to distally advance a stack of clips and to advance a distal-most clip of the stack of clips into a clip track in the first and second jaws.

## Description

### FIELD

Surgical clip appliers and methods for using the same are provided for ligating tissue, such as vessels, other ducts, and the like.

### BACKGROUND

Surgical clip appliers are commonly used for ligating a blood vessel, a duct, shunt, or a portion of body tissue during surgery. Most clip appliers typically have a handle with an elongate shaft having a pair of movable opposed jaws formed on an end thereof for holding and forming a ligation clip therebetween. The jaws are positioned around the vessel or duct in, and the clip is crushed or formed on the vessel by the closing of the jaws.

Visibility during use of a clip applier can be important for proper positioning of a clip. Any lack of visibility can lead to various opportunities for user error, including, for example, incorrect clip positioning, inadequate clip size selection, and accidental damage to the anatomy during surgery.

Accordingly, despite existing technologies, there remains a need for improved devices and methods for ligating tissue, such as vessels, ducts, and the like.

### SUMMARY

Surgical clip appliers and methods for using the same are provided herein.

In one exemplary embodiment, a surgical clip applier is provided and can include an elongate shaft, first and second jaws on a distal end of the elongate shaft, and a clip advancing assembly extending through the elongate shaft. The first and second jaws can be configured to move between open and closed positions for engaging tissue therebetween, and each jaw can have a clip engaging portion with inward facing surfaces defining a clip track for receiving and guiding a clip into the jaws. The first jaw can have an extended tip portion extending distally beyond the clip engaging portion such that the first jaw has a length that is greater than a length of the second jaw. The device can also include a clip forming assembly extending through the elongate shaft and configured to move the jaws from the open position to the closed position, and a clip advancing assembly extending through the elongate shaft and configured to distally advance a distal-most clip in a stack of clips disposed within the elongate shaft into the clip track in the first and second jaws.

The jaws can have a variety of configurations. In one embodiment, the clip track on the first jaw can terminate at a junction between the clip engaging portion and the extended tip portion. In another embodiment, at least a portion of the extended tip portion of the first jaw can extend at a transverse angle relative to the clip engaging portion of the first jaw. For example, at least a portion of the extended tip portion of the first jaw can extend at an angle greater than 0 degrees and less than 90 degrees relative to the clip engaging portion of the first jaw. In another example, at least a portion of the extended tip portion can extend at an angle in the range of about 12° to 65° relative to the clip engaging portion of the first jaw.

In one aspect, the extended tip portion can have at least a width that decreases in a distal direction such that the extended tip portion is tapered distally. In other aspects, the clip engaging portion of the first jaw can be formed of a first material having a first modulus of elasticity value and the extended tip portion can be formed of a second material having a second modulus of elasticity value that is less than the first modulus. In another aspect, a ratio of the length of the first jaw to the length of the second jaw can be in the range of about 1.1 to 1.75. In one aspect, the extended tip portion of the first jaw can be of a color that is different than a color of the clip engaging portion of the first jaw. In another aspect, a distal-most end of the extended tip portion of the first jaw can include a ball-shaped feature formed thereon.

In another embodiment, a surgical clip applier is provided and can include an elongate shaft having a jaw assembly at a distal end thereof. The jaw assembly can include opposed first and second jaws movable between open and closed positions for engaging tissue therebetween in which the first and second jaws define a clip engaging portion therebetween for receiving a clip. The first jaw can have a tip extension that extends distally beyond a distal-most end of the clip engaging portion at a transverse angle relative to the clip engaging portion. The surgical clip applier can also include a clip advancing assembly disposed in the elongate shaft. The clip advancing assembly can be configured to advance a plurality of clips distally through the elongate shaft and to advance a distal-most clip into the clip engaging portion between the first and second jaws.

In one embodiment, the tip extension of the first jaw can be more flexible than the clip engaging portion of the first and second jaws. In other aspects, at least a portion of the tip extension can extend at an angle greater than 0 and less than 90 degrees relative to the clip engaging portion. In one aspect, the tip extension can have a width, height, or both that decreases in a distal direction such that the tip extension is tapered distally. In one embodiment, the tip extension of the first jaw can be of a color that is different than a color of the clip engaging portion of the first and second jaws. In another embodiment, the first and second jaws can each include upper and lower rails extending longitudinally along inward facing surfaces thereof in which the upper and lower rails define the clip engaging portion. In other aspects, a distal end of the tip extension can include a ball-shaped feature thereon.

Methods for applying a surgical clip to tissue are also provided. In one embodiment, the method can include positioning tissue between first and second jaws on a distal end of a shaft of a clip applier, with the first jaw being positioned adjacent to a distal side of the tissue and the second jaw being positioned adjacent to a proximal side of the tissue. The method can also include actuating the clip applier or robotically / telemanipulator controlled to approximate the first and second jaws. The first and second jaws can have a clip therebetween that deforms to engage the tissue between the first and second jaws. The first jaw can have a tip extension that extends distally beyond a distal-most end of the second jaw such that the tip extension is at least partially visible when the first jaw is positioned adjacent to the distal side of the tissue and during actuation of the clip applier.

In one embodiment, the tip extension of the first jaw can have a color that differs from a remainder of the first jaw and from the second jaw to facilitate visualization of the tip extension. In another embodiment, prior to actuating the clip applier, the method can include manipulating the tip extension to dissect the tissue.

### BRIEF DESCRIPTION OF THE DRAWINGS

This invention will be more fully understood from the following detailed description taken in conjunction with the accompanying drawings, in which:
FIG. 1 is a side view of one exemplary embodiment of a surgical clip applier;
FIG. 2 is an exploded view of a distal portion of the surgical clip applier of FIG. 1;
FIG. 3 is a perspective view of a distal portion of the surgical clip applier of FIG. 1;
FIG. 4A is a perspective, partially transparent view of a proximal portion of the surgical clip applier of FIG. 1;
FIG. 4B is another perspective view of the proximal portion of the surgical clip applier of FIG. 1;
FIG. 5A is a top view of a preformed clip that can be disposed between the jaws of the surgical clip applier of FIG. 1;
FIG. 5B is a top view of a formed clip engaged with tissue;
FIG. 6A is a perspective view of a distal portion of a surgical clip applier, in which one of the opposed jaws includes an extended tip portion;
FIG. 6B is a top view of the distal portion of the surgical clip applier of FIG. 6A;
FIG. 7 is a top view of a distal portion of another embodiment of a surgical clip applier having a ball-shaped feature at a distal-most end of an extended tip portion of a jaw; and
FIG. 8 is a side perspective view of the surgical clip applier of FIGS. 6A and 6B, showing tissue positioned between the jaws, with the first jaw placed adjacent to the distal side of the tissue and the second jaw placed adjacent to the proximal side of the tissue.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

It will be appreciated that the terms "proximal" and "distal" are used herein with reference to a user, such as a clinician, gripping a handle of an instrument. Other spatial terms such as "front" and "rear" similarly correspond respectively to distal and proximal. It will be further appreciated that for convenience and clarity, spatial terms such as "vertical" and "horizontal" are used herein with respect to the drawings. However, surgical instruments are used in many orientations and positions, and these spatial terms are not intended to be limiting and absolute.

Surgical clip appliers and methods for applying surgical clips to a vessel, duct, etc., during a surgical procedure are provided. The surgical clip appliers generally include opposed first and second jaws on the distal end of an elongate shaft, and the first jaw can have an extended tip such that a length of the first jaw that is greater than a length of the second jaw. Such a configuration can improve visibility of the first jaw during placement and actuation of the clip applier. The extended tip can also aid in tissue dissection. An exemplary surgical clip applier can include a variety of features to facilitate application of a surgical clip, as described herein and illustrated in the drawings. However, a person skilled in the art will appreciate that the surgical clip applier can include only some of these features and/or it can include a variety of other features known in the art. The surgical clip applier described herein is merely intended to represent certain exemplary embodiments.

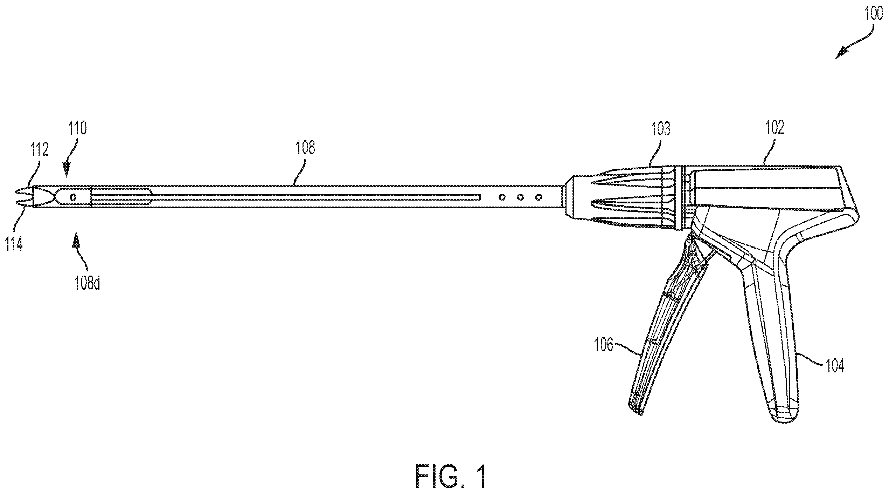

FIGS. 1-4B illustrate one embodiment of a surgical clip applier 100. As shown, the surgical clip applier 100 generally includes a housing 102 having a stationary handle 104 and a movable handle or trigger 106 that is pivotally coupled to the housing 102. An elongate shaft 108 extends distally from the housing 102 and includes a jaw assembly 110 formed on a distal end 108d thereof and including first and second jaws 112, 114 that are movable between open and closed positions. The first and second jaws 112, 114 include opposed inward facing surfaces and each inward facing surface has a clip track formed therealong for receiving and guiding legs of a preformed clip 127 into the first and second jaws 112, 114. The elongate shaft 108 can be rotated with respect to the housing 102 via a rotation knob 103.

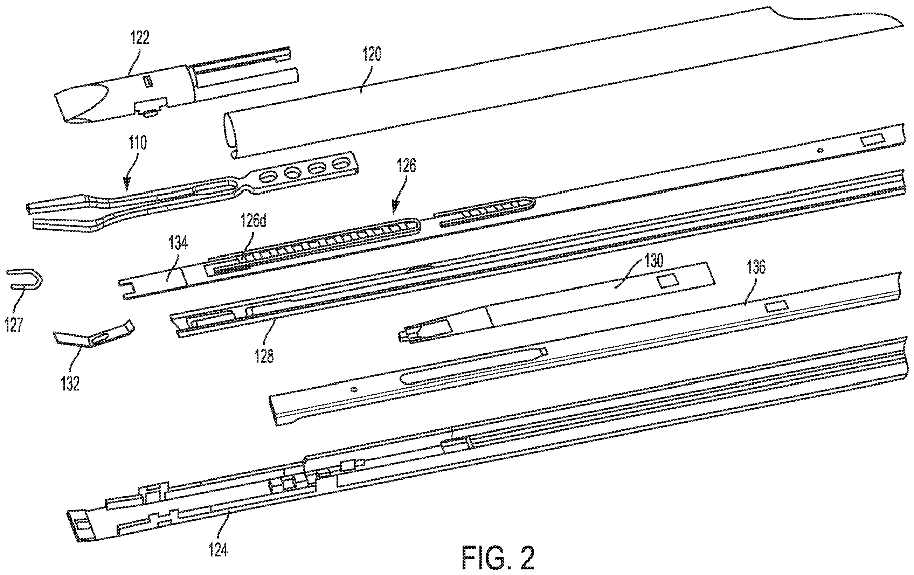

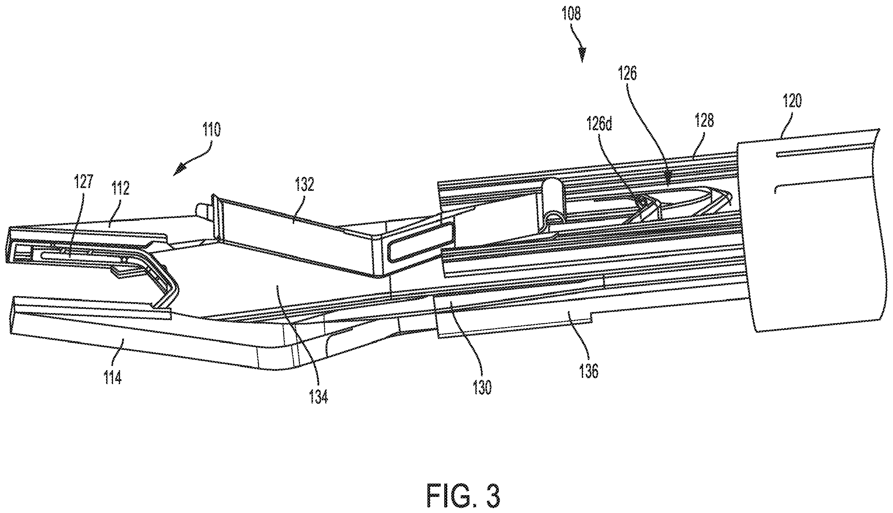

As shown in FIGS. 2 and 3, the elongate shaft 108 can include an outer support tube 120, an upper shroud 122 coupled distally to the outer tube 120, and a lower shroud 124. The outer support tube 120 and the upper and lower shrouds 122, 124 form an outer casing of the shaft 108. As shown in FIGS. 2 and 3, a clip stack 126 including multiple surgical clips is disposed within a clip track or holder 128 of the shaft 108 proximal to the first and second jaws 112, 114, and is biased distally. A floor 130 extends beneath the clip stack 126 for maintaining the clip stack 126 in alignment within the shaft 108, and for guiding a distal-most clip 126d into the jaws 112, 114. A lifter spring 132 is positioned just proximal to the jaws 112, 114 and distal to the clip stack 126 for preventing distal movement of the clip stack 126, with the distal-most clip 126d disposed around the lifter spring 132. A feeder bar 134 extends through the elongate shaft 108 for feeding the distal-most clip 126d into the jaws. As shown in FIG. 3 illustrating the clip applier 100 with the upper and lower shrouds 122, 124 removed, a former tube 136 extends around a proximal end of the jaws 112, 114 and is movable distally to cam the jaws 112, 114 to a closed position for deforming a clip 127 disposed therebetween.

While the outer support tube 120 of the elongated shaft 108 can have a variety of configurations, in this exemplary embodiment, as shown in FIGS. 2-3, the outer tube 108 have a cylindrical-tube configuration. In other embodiments, the elongate shaft can have a square-tube configuration. Thus, the elongate shaft 108 can be shaped for use in laparoscopic or open clip appliers.

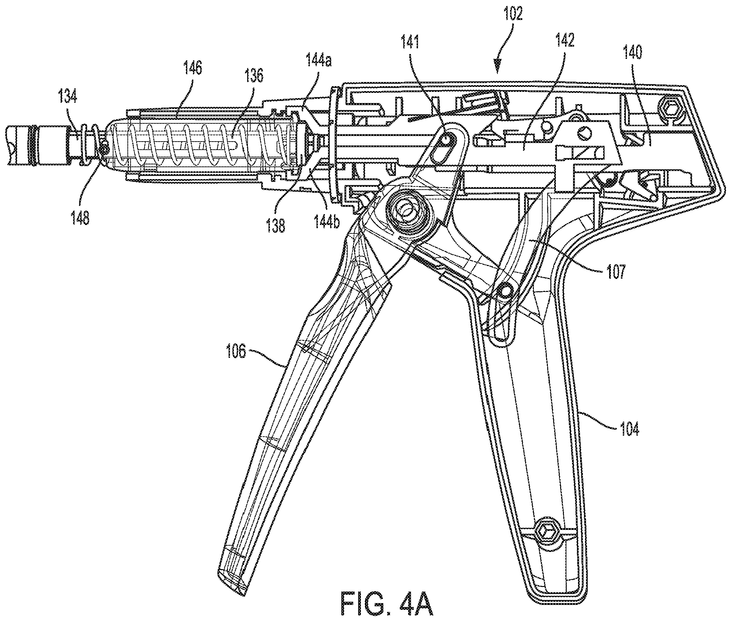

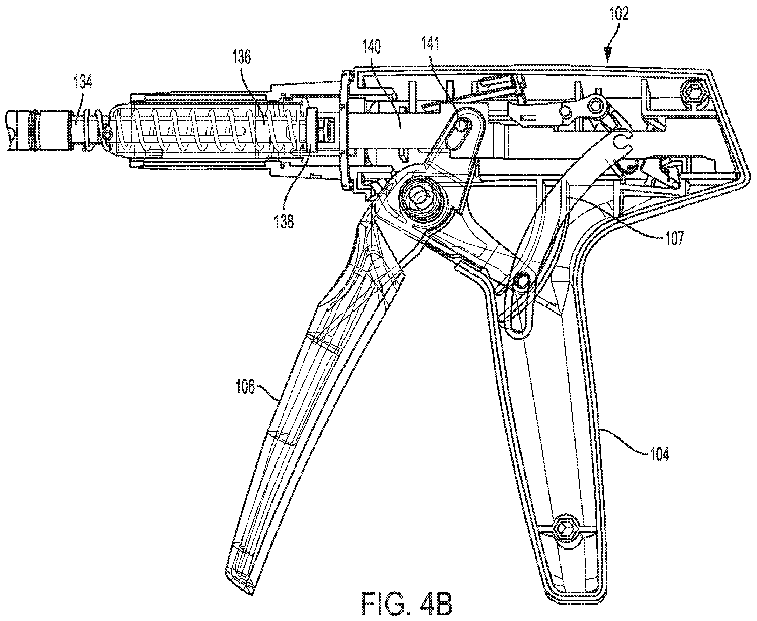

The surgical clip applier 100 has a clip forming assembly including various components that operate together to close the jaws 112, 114 when the trigger 106 is activated to thereby cause a preformed clip (e.g., clip 127) disposed in the jaws to be applied (formed) to the tissue. The clip forming assembly encompasses the former tube 136 and other components that are coupled to the trigger 106 configured to be activated to move the former tube 136 distally to thereby close the jaws 112, 114. A clip advancing assembly of the surgical clip applier 100 includes the feeder bar 134 that is also coupled to the trigger 106, via a link 107 extending proximally from the trigger 106, as shown in FIGS. 4A and 4B. In this way, when the trigger 106 is activated, the feeder bar 134 is caused to move proximally, opposite to a distal direction in which the former tube 136 is moved upon activation of the trigger 106.

The clip forming and clip advancing assemblies can have any suitable configurations. For example, in the illustrated embodiment, as shown in FIGS. 4A and 4B, the former tube 136 of the clip forming assembly is coupled, via an inner coupling 138, to a former plate 140 in the handle 102 that is, in turn, coupled to the trigger 106 via a pin 141, and the feeder bar 134 of the clip advancing assembly is coupled to the trigger 106 via a feeder plate 142 that is also coupled to the trigger 106, via the link 107. As shown in FIG. 4A, the feeder plate 142 has arms 144a, 144b at a distal end thereof that are disposed over and mate with a proximal end of an outer coupling 146 (shown partially transparent). A connecting pin 148 at a distal end of the outer coupling 146 attaches the feeder bar 134 to the outer coupling 146. FIGS. 4A and 4B illustrate the handle 102 with part of an outer casing removed, and FIG. 4B shows the handle 102 without the feeder plate 142, for illustration purposes only. It should be appreciated that the surgical clip applier 100 can include various other components and assemblies that are not described herein for the sake of simplicity.

A person skilled in the art will appreciate that, while a trigger is shown and described, the clip appliers disclosed herein need not include a trigger, and can have a variety of other actuation mechanisms. For example, the clip applier can be powered and can include an actuation button for actuating a motor to control firing of the device. In other embodiments, the housing can be configured to couple to a robotic system, such that actuation of the device is controlled through the robotic / telemanipulator system.

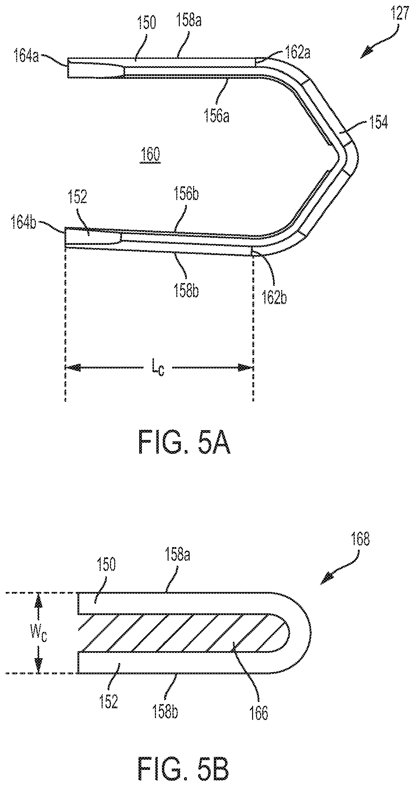

In use, when the trigger 106 of the handle 102 is activated (e.g., moved towards the stationary handle 104), the former plate 140 of the clip forming assembly is advanced distally to cause the former tube 136 to advance distally over the jaws 112, 114, thereby camming the jaws 112, 114 to the closed position. At the same time, the feeder plate 142 of the clip advancing assembly is moved proximally, thereby pulling the feeder bar 134 proximally to position the feeder bar 134 proximal of the distal-most clip 126d of the clip stack 126. Once the preformed clip 127, disposed in the jaws 112, 114 such that clip's legs are received within the clip track of each of the jaws, is fully formed into a formed clip, the trigger 106 is released, which causes the clip forming assembly to move proximally while the clip advancing assembly moves distally. The original, preformed clip 127 is shown in more detail in FIG. 5A. The proximal movement of the clip forming assembly causes the former tube 136 to retract relative to the jaws, thus allowing the jaws 112, 114 to move to the original open position, thereby releasing the formed clip 168, which is shown in more detail in FIG. 5B. The distal movement of the clip advancing assembly causes the feeder bar 134 to move distally, and the feeder bar 134 thereby pushes the distal-most clip 126d distally, overcoming the biasing force of the lifter spring 132 and causing the lifter spring 132 to deflect out of the way, thereby allowing the distal-most clip 126d to be advanced into the jaws 112, 114. In this way, the distal-most clip becomes positioned in the jaws' clip tracks, like the clip 127 in FIG. 3. The floor 130 helps guide the distal-most clip into the clip tracks of the jaws 112, 114.

As shown in FIG. 5A, the original, preformed clip 127 can have opposing first and second legs 150, 152 and an intermediate portion 154 extending therebetween. The first and second legs 150, 152 can each be defined by their respective inner facing surfaces 156a, 156b and outer facing surfaces 158a, 158b in which the inner facing surfaces 156a, 156b define a tissue engaging portion 160. The first and second legs 150, 152 can each distally extend from their respective proximal end 162a, 162b to distal end 164a, 164b. The length (L_{c}) of the original, preformed clip 127 can be defined by the length of either legs 150, 152. As discussed above, when the original, preformed clip 127 is deformed about tissue 166, via the former tube 136 moving distally to close the jaws 112, 114, the tissue 166 can be engaged within the tissue engaging portion 160, resulting in a formed clip 168 as shown in FIG. 5B. The width (W_{c}) of the formed clip 168 is the distance that laterally extends from the outer surface 158a of the first leg 150 to the outer surface 158b of the second leg 152, including tissue 166 engaged therebetween.

Generally, during use, one of the opposed jaws of the surgical clip applier, i.e., the rear jaw, is placed adjacent to the distal side of tissue, as described in more detail below. As a result, the rear jaw can be obstructed by the tissue due to camera angle during a surgical procedure. This lack of visibility of the rear jaw can lead to incorrect clip positioning, inadequate clip size selection, and/or accidental damage to the anatomy during surgery. Accordingly, various embodiments of a pair of opposing jaws are described below in which the jaws are configured to increase the visibility of the rear jaw during positioning of the opposed jaws about tissue and actuation of the surgical clip applier. In general, the pair of opposed jaws include a first jaw having a length that is greater than a length of a second jaw. The opposed jaws can be movable between open and closed positions to deform a preformed clip located therebetween so as to engage tissue positioned between the pair of opposed jaws. The extended length of the first jaw, as compared to the length of the second jaw, can provide visibility benefits not otherwise possible with conventional opposing jaw configurations (i.e., opposing jaws that are of equal length), thereby improving efficiency and effectiveness of the surgical clip applier during a surgical procedure. Furthermore, unlike conventional opposing jaws, the extension of the first jaw can also be used for dissection of tissue without dislodging the preformed clip disposed within the jaws, which results in greater access to the tissue, and therefore more accurate positioning and placement of the clip about the tissue.

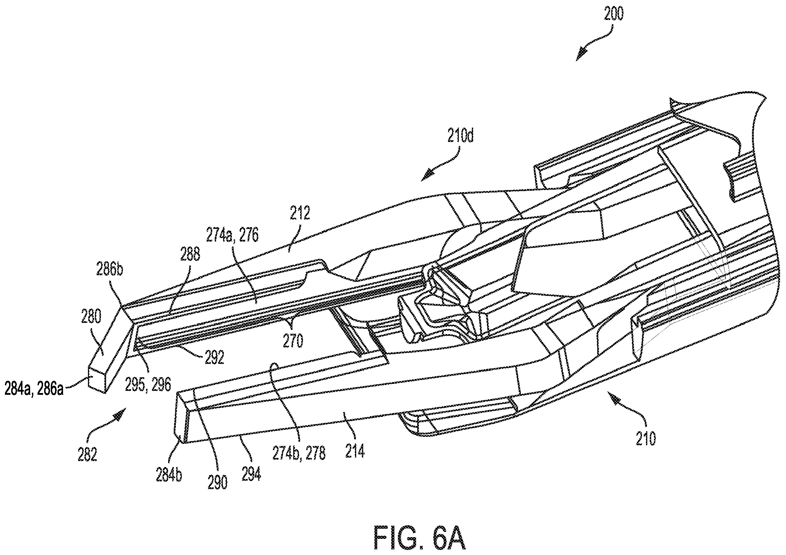

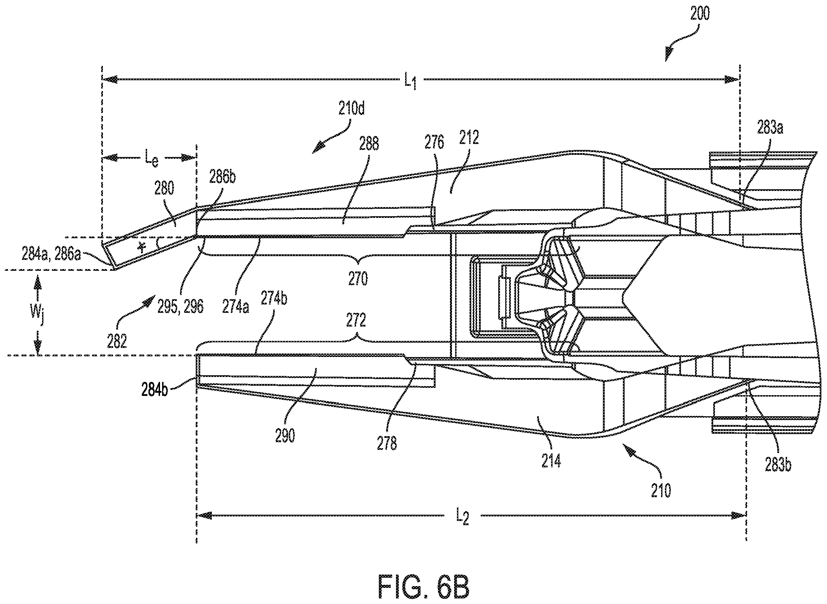

In general, the jaw assembly can include a proximal portion that extends into the elongated shaft of the surgical clip applier and a distal portion that splits into two parts that form a pair of opposing jaws. FIGS. 6A and 6B illustrate a distal portion 210d of an exemplary embodiment of a jaw assembly 210 of a surgical clip applier 200. The surgical applier 200 can be similar to surgical clip applier 100 (FIGS. 1-4B) and is therefore not described in detail herein. As shown, the distal portion 210d of the jaw assembly 210 is located at a distal end of the surgical clip applier 200. While the jaw assembly 210 can have a variety of configurations, the jaw assembly 210, as shown in FIGS. 6A and 6B, includes first and second jaws 212, 214 each having a clip engaging portion 270, 272 with inward facing surfaces 274a, 274b defining clip tracks 276, 278. The clip tracks 276, 278 can be sized and configured for receiving and guiding an original, preformed clip, like clip 127 shown in FIG. 5A, into the jaw assembly 210.

As shown in FIGS. 6A-6B, the clip engaging portions 270, 272 can be defined by the upper rails 288, 290 and the lower rails 292, 294 of the first and second jaws 212, 214. As previously mentioned, the inward facing surfaces 274a, 274b of the clip engaging portions 270, 272 can each define a clip track 276, 278. The clip tracks 276, 278 can be adapted for receiving and guiding a distal-most, preformed clip into the jaw assembly 210. For example, when the clip advancing assembly is actuated, the distal-most, preformed clip, like clip 127 in FIG. 5A, is distally advanced into the clip tracks 276, 278 in the first and second jaws 212, 214, thereby positioning the preformed clip, like clip 127, therebetween. While the clip tracks 276, 278 can have a variety of configurations, in this exemplary embodiment, the clip track 276 on the first jaw 212 terminates at a junction 296, which is located at a distal-most edge 295 of the inward facing surface 274a. In this exemplary embodiment, the clip tracks 276,278 are closed-edged clip tracks. In other embodiments, the clip tracks 276. 278 can be open clip tracks in which the upper and lower rails 288, 292 of the first jaw 212 extends to the proximal end 286b of the extended tip portion 280, and the upper and lower rails 290, 294 extended to the distal end 284b of the second jaw 214.

As further shown in FIGS. 6A and 6B, a jaw opening 282 can be defined between the distal end 284a of the first jaw 212 and the distal end 284b of the second jaw 214. During use of the surgical clip applier 200, the jaw opening 282 can help guide tissue between the first and second jaws 212, 214. While the width (Wⱼ) of the jaw opening 282 can vary depending at least on the configuration of the first jaw, in exemplary embodiments, the width (Wj) of the jaw opening 282 can be greater than or equal to the width (W_{c}) of a formed clip, like 168 shown in FIG. 5B.

Further, the first jaw 212 can have an extended tip portion 280 that extends distally beyond its clip engaging portion 270. While the extended tip portion 280 can have various shapes and sizes to aid in proper placement of the jaws assembly 210 about the tissue of interest during surgery, in some implementations, the extended tip portion 280 can be tapered distally. For example, as shown in FIGS. 6A-6B, the extended tip portion 280 can have a cross-sectional width, height, or both that decreases in a distal direction such that the extended tip portion 280 is tapered distally. This tapered structure can improve accuracy of the surgical clip applier 200 when dissecting tissue and minimize accidental damage to the tissue itself and other surrounding tissue. The cross-sectional shape of the extended tip portion 280 can also vary, and it can be square, rectangular, circular, ovular, etc.

The extended tip portion 280 can also extend at a various angles relative to the clip engaging portion 270 of the first jaw 212. In the illustrated embodiment, the extended tip portion 280 extends at a transverse angle ( 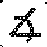 ) that is greater than 0 degrees and less than 90 degrees relative to the clip engaging portion 270 of the first jaw 212. In certain exemplary embodiment, the extended tip portion 280 can extend at an angle in the range of about 0° to 90° relative to the clip engaging portion 270 of the first jaw 212. In other embodiments, the angle can be about 12° to 65°, about 20° to 25°, or about 45° to 65°. The extended tip portion 280 can extend at an angle relative to the clip engaging portion 270 of the first jaw 212 between any of these recited angles. In one embodiment, the extended tip portion 280 can be in the form of a curve relative to the clip engaging portion 270 of the first jaw, or in other embodiments, the extended tip portion 280 can be in the form of other suitable shapes. It is also contemplated that the extended tip portion 280 is co-linear with the clip engaging portion 270 of the first jaw 212, and therefore does not extend at a transverse angle. It is also contemplated that a portion of the extended tip portion 280 extends at a transverse angle relative the clip-engaging portion 270 of the first jaw 212, and therefore the extended tip portion can be a non-linear shape, such as a curved shape.

The extended tip portion 280 can also have various lengths, but in some exemplary embodiments, the length of the extended tip portion 280 can be less than the length of an original, preformed clip, like clip 127 in FIG. 5A. The length (Lₑ) of the extended tip portion 280 is measured from its proximal end 286b to its distal end 286a along an axis substantially parallel to the inward facing surface 274a of the first jaw 212. In other embodiments, the extended tip portion 280 can have a length that is equal to the length of a preformed clip, like clip 127. In one embodiment, the ratio of the length of the extended tip portion 280 to the length of a preformed clip, like clip 127, can be in a range of about 0.15:1 to about 1:1. In another embodiment, the ratio of the length of the extended tip portion 280 to the length of a preformed clip, like clip 127, can be in a range of about 0.5:1 to about 1:1.5. In yet another embodiment the ratio of the length of the extended tip portion 280 to the length of a preformed clip, like clip 127, can be in a range of about 0.15:1 to 1:1.5.

It should be noted that the structural design of the extended tip portion, e.g., length and/or extension angle, for a clip applier is preferably balanced with the need for a suitable jaw opening that can guide tissue between the first and second jaws and that can release a formed clip from the device. Unlike other surgical devices, such as surgical staplers, the distance between the first and second jaws is correlated to the width of the preformed clip so that the preformed clip can be advanced and held between the jaws until the clip is formed about tissue and then released. As such, suitable jaw openings of clip appliers can be limited.

Furthermore, it should also be noted that it can be beneficial for the clip engaging portions 270, 272 of the jaw assembly 210 to have a balance of flexibility and strength such that the jaws 212, 214 having sufficient strength to engage tissue and deform a clip therearound, while having some flexibility to prevent deformation of the jaws 212, 214. This same balance can be shared with the extended tip portion 280, or the extended tip portion 280 can vary relative to the remainder of the jaws 212, 214.

In one embodiment, the extended tip portion 280 can be formed of a different material than that of the clip engaging portion 270 of the first jaw 212. For example, the material of the extended tip portion 280 can have a durometer or modulus of elasticity value that is less (more flexible) than a durometer or modulus of elasticity value of a material of the clip engaging portion 270 of the first jaw 212. As previously mentioned, the extended tip portion 280 can be used for dissecting tissue prior to actuation of the surgical clip applier 200, and when the extended tip portion 280 is formed of a softer (more flexible) material, a user can perform atraumatic dissection without having the preformed clip prematurely dislodge from the jaws assembly 210. Moreover, with other surgical devices, such as surgical staplers, flexible extended tip portions may be undesirable because of the structural stability needed to effectively operate the end effectors. In one embodiment, the clip engaging portion 270 of the first jaw 212 can be formed of a first material having a first durometer or modulus value and the extended tip portion 280 can be formed of a second material having a second durometer or modulus value that is less than the first durometer or modulus value. In an exemplary embodiment, the second durometer value can be in a range of about Shore 20A to Shore 60A. Suitable non-limiting examples of the second material include plastics or elastomers. In other exemplary embodiments, the first modulus value can be from about 50 GPa to 220 GPa and the second modulus value can be from about 0.001 GPa to 20 GPa. The first material and second material of the first jaw can have a first modulus and a second modulus, respectively, between any of these recited ranges.

In another embodiment, the material of the extended tip portion 280 can have a durometer or modulus of elasticity value that is greater (harder) than a durometer or modulus of elasticity value of a material of the clip engaging portion 270 of the first jaw 212. For example, in one embodiment, the clip engaging portion 270 of the first jaw 212 can be formed of a first material having a first durometer or modulus value and the extended tip portion 280 can be formed of a second material having a second durometer or modulus value that is greater than the first durometer value.

In other embodiments, the extended tip portion 280 can include a base material and a second material that is overmolded onto and disposed over the base material and that is formed of a material that is different than the base material. In some embodiments, the overmold material can be less stiff (more flexible) than the base material. For example, the base material, which can be formed of the same material as the clip engaging portion 270 of the first jaw 212, can have a first durometer or modulus of elasticity value and the overmold material can have a second durometer or modulus of elasticity value that is less than the first durometer or modulus value. In an exemplary embodiment, the overmold material can have a durometer value in a range of about Shore 20A to Shore 80A. Suitable non-limiting examples of the overmold material include plastics or elastomers. In other exemplary embodiments, the base material can have a modulus value from about 50 GPa to 220 GPa and the overmold material can have a modulus value from about 0.001 GPa to 20 GPa. The base material and overmold material of the first jaw of this disclosure also may have a first modulus and a second modulus, respectively, between any of these recited ranges.

In another embodiment, the overmold material can be more stiff (harder) than the base material. For example, the base material, which can be formed of the same material as the clip engaging portion 270 of the first jaw 212, can have a first durometer or modulus of elasticity value and the overmold material can have a second durometer or modulus of elasticity value that is greater than the first durometer or modulus value.

To further assist a user in identifying the first jaw 212 when tissue is positioned between the first and second jaws 212, 214 during surgery, in other embodiments, the extended tip portion 280 of the first jaw 212 can be of a color that is different than a color of the clip engaging portion 270 of the first jaw 212. For example, in some implementations, the extended tip portion 280 can be yellow or green. Alternatively, or in addition to color, the extended tip portion 280 can include other features, such as markings, to aid in the identification of the first jaw 212. As a result, when the first jaw 212 is positioned adjacent to the distal side of the tissue, the extended tip portion 280 can be more readily visible to the user, thereby improving efficiency and effectiveness of the surgical clip applier 200 during a surgical procedure.

Given that the first jaw 212 includes the extended tip portion 280, the first jaw 212 will thus have a length (L₁) that is greater than a length (L₂) of the second jaw 214. The lengths of both the first jaw 212 and the second jaw 214 each extend from their corresponding proximal ends 283a, 283b to their corresponding distal ends 284a, 284b as shown in FIGS. 6A and 6B. The length of the first jaw 212 can vary so long as the length of the first jaw 212 is greater than the length of the second jaw 214. In one embodiment, the ratio of the length of the first jaw 212 to the length of the second jaw 214 can be in the range of about 1.1 to 1.75. In another embodiment, the ratio of the length of the first jaw 212 to the length of the second jaw 214 can be in the range of about 1.1 to 1.25 or about 1.5 to about 1.75. The jaw assembly of this disclosure may have a ratio of the length of first jaw to the length of the second jaw any of these recited ratio. As discussed above, the extended length of the first jaw 212 can advantageously increase the visibility of the first jaw 212 during a surgical procedure in which the first jaw 212 is placed behind the tissue of interest, and/or can facilitate with tissue dissection.

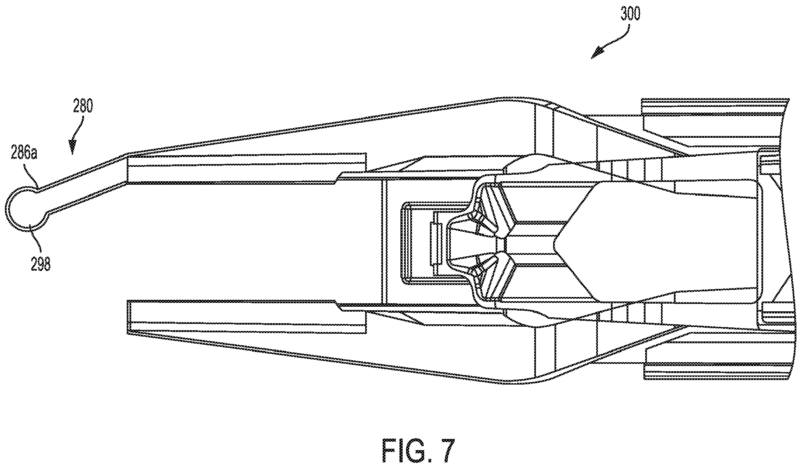

The extended tip portion 280 can also include other features that can assist with dissecting tissue. For example, as illustrated in FIG. 7, which is another exemplary embodiment of a distal portion of a surgical clip applier 300 that is similar to the surgical clip 200 (FIGS. 6A and 6B), the distal-most end 286a of the extended tip portion 280 can include a ball-shaped feature 298. This ball-shaped feature 298 can assist with blunt dissection of tissue. It is also contemplated that the extended tip portion 280 can include alternative shaped features, such as a triangulated-shaped feature, or additional features to minimize damage to the dissected tissue and/or other surrounding tissue. It is also contemplated that the extended tip portion 280 can include localized blunt serrations or gripping features, such as the grasping type features of the babcock or maryland graspers, to facilitate gross dissection while not inducing acute tissue damage.

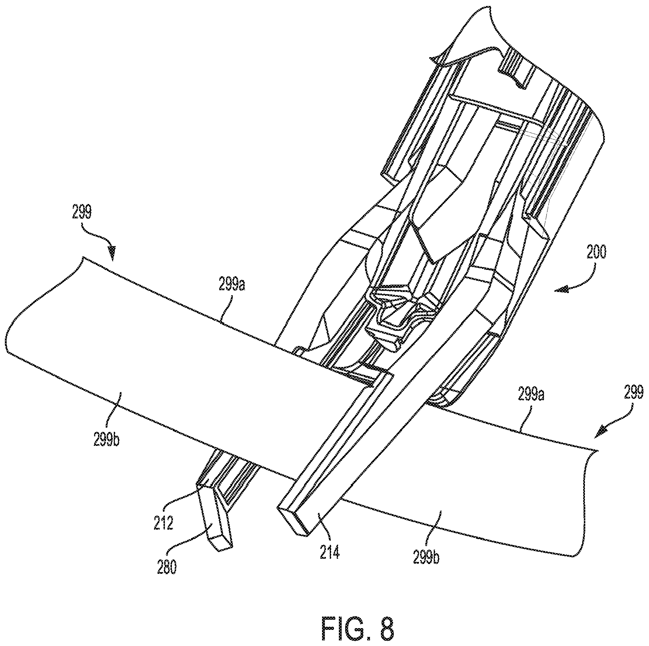

As previously mentioned, the surgical clip applier can be used to form a clip about a surgical site, such as a vessel, duct, shunt, etc. FIG. 8 illustrates the surgical clip applier 200 (FIGS. 6A-6B), in use, when tissue 299 is positioned between the first and second jaws 212, 214. Prior to positioning of the tissue 299, a user can manipulate the extended tip portion 280 of the first jaw 212 to dissect the tissue 299. As shown in FIG. 8, the first jaw 212 can be positioned adjacent to a distal side 299a of the tissue 299 in which the extended tip portion 280 is at least partially visible, and the second jaw 214 can be positioned adjacent to a proximal side 299b of the tissue 299. Once the jaws 212, 214 are positioned about the tissue 299, the trigger (or other actuation device) of the surgical clip applier 200 can be actuated to cause the distal-most, preformed clip, like clip 127 shown in FIG. 5A, to be advanced from the clip advancing assembly into the jaws 212, 214, and to approximate the first and seconds jaws 212, 214 such that the preformed clip, like clip 127, deforms and engages the tissue 299 between the jaws 212, 214, thereby resulting in a formed clip, like clip 168 shown in FIG. 5B.

The following is a non-exhaustive list of embodiments of the invention that may or may not be claimed.
1. A surgical clip applier, comprising:
   an elongate shaft;
   first and second jaws on a distal end of the elongate shaft and configured to move between open and closed positions for engaging tissue therebetween, the first and second jaws each having a clip engaging portion with inward facing surfaces defining a clip track for receiving and guiding a clip into the jaws, and the first jaw having an extended tip portion extending distally beyond the clip engaging portion such that the first jaw has a length that is greater than a length of the second jaw;
   a jaw forming assembly extending through the elongate shaft and configured to move the jaws from the open position to the closed position; and
   a clip advancing assembly extending through the elongate shaft and configured to distally advance a distal-most clip in a stack of clips disposed within the elongate shaft into the clip track in the first and second jaws.
2. The surgical clip applier of embodiment 1, wherein the clip track on the first jaw terminates at a junction between the clip engaging portion and the extended tip portion.
3. The surgical clip applier of embodiment 1, wherein at least a portion of the extended tip portion extends at a transverse angle relative to the clip engaging portion of the first jaw.
4. The surgical clip applier of embodiment 1, wherein at least a portion of the extended tip portion extends at an angle greater than 0 degrees and less than 90 degrees relative to the clip engaging portion of the first jaw.
5. The surgical clip applier of embodiment 1, wherein at least a portion of the extended tip portion extends at an angle in the range of about 12 degrees to 65 degrees relative to the clip engaging portion of the first jaw.
6. The surgical clip applier of embodiment 1, wherein the extended tip portion has a width, height, or both that decreases in a distal direction such that the extended tip portion is tapered distally.
7. The surgical clip applier of embodiment 1, wherein the clip engaging portion of the first jaw is formed of a first material having a first modulus of elasticity value and the extended tip portion is formed of a second material having a second modulus of elasticity value that is less than the first modulus of elasticity value.
8. The surgical clip applier of embodiment 1, wherein a ratio of the length of the first jaw to the length of the second jaw is in the range of about 1.1 to 1.75.
9. The surgical clip applier of embodiment 1, wherein the extended tip portion of the first jaw is of a color that is different than a color of the clip engaging portion of the first jaw.
10. The surgical clip applier of embodiment 1, wherein a distal-most end of the extended tip portion includes a ball-shaped feature or a triangulated-shaped feature formed thereon.
11. A surgical clip applier, comprising:
   an elongate shaft having a jaw assembly at a distal end thereof, the jaw assembly including opposed first and second jaws movable between open and closed positions for engaging tissue therebetween, the first and second jaws defining a clip engaging portion therebetween for receiving a clip, and the first jaw having a tip extension that extends distally beyond a distal-most end of the clip engaging portion at a transverse angle relative to the clip engaging portion; and
   a clip advancing assembly disposed in the elongate shaft and configured to advance a plurality of clips distally through the elongate shaft and to advance a distal-most clip into the clip engaging portion between the first and second jaws.
12. The surgical clip applier of embodiment 11, wherein the tip extension of the first jaw is more flexible than the clip engaging portion of the first and second jaws.
13. The surgical clip applier of embodiment 11, wherein at least a portion of the tip extension extends at an angle greater than 0 and less than 90 degrees relative to the clip engaging portion.
14. The surgical clip applier of embodiment 11, wherein the first and second jaws each include upper and lower rails extending longitudinally along inward facing surfaces thereof, the upper and lower rails defining the clip engaging portion.
15. The surgical clip applier of embodiment 11, wherein the tip extension has a width, height, or both that decreases in a distal direction such that the tip extension is tapered distally.
16. The surgical clip applier of embodiment 11, wherein the tip extension of the first jaw is of a color that is different than a color of the clip engaging portion of the first and second jaws.
17. The surgical clip applier of embodiment 11, wherein a distal end of the tip extension includes a ball-shaped feature thereon.
18. A method for applying a surgical clip to tissue, comprising:
   positioning tissue between first and second jaws on a distal end of a shaft of a clip applier, the first jaw being positioned adjacent to a distal side of the tissue and the second jaw being positioned adjacent to a proximal side of the tissue;
   actuating the clip applier to approximate the first and second jaws, the first and second jaws having a clip therebetween that deforms to engage the tissue between the first and second jaws;
   wherein the first jaw has a tip extension that extends distally beyond a distal-most end of the second jaw such that the tip extension is at least partially visible when the first jaw is positioned adjacent to the distal side of the tissue and during actuation of the clip applier.
19. The method of embodiment 18, wherein the tip extension of the first jaw has a color that differs from a remainder of the first jaw and from the second jaw to facilitate visualization of the tip extension.
20. The method of embodiment 18, further comprising, prior to actuating the clip applier, manipulating the tip extension to dissect the tissue.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended claims.

## Claims

1. A surgical clip applier, comprising:
an elongate shaft;
first and second jaws on a distal end of the elongate shaft and configured to move between open and closed positions for engaging tissue therebetween, the first and second jaws each having a clip engaging portion with inward facing surfaces defining a clip track for receiving and guiding a clip into the jaws, and the first jaw having an extended tip portion extending distally beyond the clip engaging portion such that the first jaw has a length that is greater than a length of the second jaw; and
a clip advancing assembly extending through the elongate shaft and configured to distally advance a distal-most clip in a stack of clips disposed within the elongate shaft into the clip track in the first and second jaws.

2. The surgical clip applier of claim 1, further comprising a jaw forming assembly extending through the elongate shaft and configured to move the jaws from the open position to the closed position.

3. The surgical clip applier of claim 1 or 2, wherein the clip track on the first jaw terminates at a junction between the clip engaging portion and the extended tip portion.

4. The surgical clip applier of any preceding claim, wherein at least a portion of the extended tip portion extends at a transverse angle relative to the clip engaging portion of the first jaw.

5. The surgical clip applier of any preceding claim, wherein at least a portion of the extended tip portion extends at an angle greater than 0 degrees and less than 90 degrees relative to the clip engaging portion of the first jaw.

6. The surgical clip applier of any preceding claim, wherein at least a portion of the extended tip portion extends at an angle in the range of about 12 degrees to 65 degrees relative to the clip engaging portion of the first jaw.

7. The surgical clip applier of any preceding claim, wherein the extended tip portion has a width, height, or both that decreases in a distal direction such that the extended tip portion is tapered distally.

8. The surgical clip applier of any preceding claim, wherein the clip engaging portion of the first jaw is formed of a first material having a first modulus of elasticity value and the extended tip portion is formed of a second material having a second modulus of elasticity value that is less than the first modulus of elasticity value.

9. The surgical clip applier of any preceding claim, wherein a ratio of the length of the first jaw to the length of the second jaw is in the range of about 1.1 to 1.75.

10. The surgical clip applier of any preceding claim, wherein the extended tip portion of the first jaw is of a color that is different than a color of the clip engaging portion of the first jaw.

11. The surgical clip applier of any preceding claim, wherein a distal-most end of the extended tip portion includes a ball-shaped feature or a triangulated-shaped feature formed thereon.

12. The surgical clip applier of any preceding claim, wherein the clip advancing assembly is configured to advance the stack of clips distally through the elongate shaft.

13. The surgical clip applier of any preceding claim, wherein the extended tip portion of the first jaw is more flexible than the clip engaging portion of the first and second jaws.

14. The surgical clip applier of any preceding claim, wherein the first and second jaws each include upper and lower rails extending longitudinally along inward facing surfaces thereof, the upper and lower rails defining the clip engaging portion.

15. The surgical clip applier of any preceding claim, wherein the extended tip portion of the first jaw is of a color that is different than a color of the clip engaging portion of the first and/or second jaws.
